(19) **Europäisches Patentamt / European Patent Office / Office européen des brevets**

(11) **EP 4 327 937 A1**

(12) # EUROPEAN PATENT APPLICATION
published in accordance with Art. 153(4) EPC

(43) Date of publication:
**28.02.2024  Bulletin 2024/09**

(21) Application number: **22791764.8**

(22) Date of filing: **20.04.2022**

(51) International Patent Classification (IPC):
**B01J 23/46** (2006.01)      **B01J 23/63** (2006.01)
**B01J 37/02** (2006.01)      **B01J 37/04** (2006.01)
**B01J 37/08** (2006.01)      **C07B 61/00** (2006.01)
**C07C 1/12** (2006.01)      **C07C 9/04** (2006.01)

(52) Cooperative Patent Classification (CPC):
**B01J 23/46; B01J 23/63; B01J 37/02; B01J 37/04; B01J 37/08; C07B 61/00; C07C 1/12; C07C 9/04; Y02P 20/133**

(86) International application number:
**PCT/JP2022/018308**

(87) International publication number:
**WO 2022/224993 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **21.04.2021  JP 2021071830**

(71) Applicant: **Osaka Gas Co., Ltd.**
**Osaka-shi, Osaka 541-0046 (JP)**

(72) Inventors:
• **OHTSUKA, Hirofumi**
  **Osaka-shi, Osaka 541-0046 (JP)**
• **NORIOKA, Shimpei**
  **Osaka-shi, Osaka 541-0046 (JP)**
• **HIRAYAMA, Akio**
  **Osaka-shi, Osaka 541-0046 (JP)**

(74) Representative: **LBP Lemcke, Brommer & Partner**
**Patentanwälte mbB**
**Siegfried-Kühn-Straße 4**
**76135 Karlsruhe (DE)**

(54) **MOLDED BODY OF CARBON DIOXIDE METHANATION CATALYST AND METHOD FOR PRODUCING THE SAME**

(57)    Provided is a molded catalyst serving as a methanation catalyst that supports ruthenium as an activated metal, and has high activity at low temperatures, sufficient strength for industrial use, and heat resistance under high temperature and high water vapor pressure conditions. Provided is a carbon dioxide methanation catalyst molded body including an activated alumina molded body, and zirconia and ruthenium supported on the activated alumina molded body, in which the amount of zirconia supported is 3 to 10 parts by mass with respect to 100 parts by mass of the activated alumina molded body, the amount of ruthenium supported is 0.1 to 5 parts by mass per 100 parts by mass of the activated alumina molded body, and the carbon dioxide methanation catalyst molded body is a molded body having a particle diameter of 2 to 20 mm.

**(Cont. next page)**

EP 4 327 937 A1

Fig.3

**Description**

Technical Field

The present invention relates to a catalyst molded body for producing a fuel gas containing methane as a main component, by reacting hydrogen and carbon dioxide, and a method for producing the same.

Background Art

In recent years, from the viewpoint of global warming countermeasures, carbon-neutral fuels, which do not substantially increase the concentration of carbon dioxide in the atmosphere even when used for combustion, have attracted attention.

Methane can be obtained by collecting carbon dioxide from exhaust gases generated by industrial processes, thermal power generation, and the like, and reacting the collected carbon dioxide with hydrogen obtained through electrolysis using power obtained through renewable energy such as solar power generation and wind power generation. The methane obtained through this method does not generate additional carbon dioxide even if it is used for combustion, and therefore it can be considered as a carbon-neutral fuel that does not affect global warming.

A methanation reaction (Formula 1) in which carbon dioxide and hydrogen are reacted to obtain methane is known.

$$CO_2 + 4H_2 \rightarrow CH_4 + 2H_2O \qquad \text{(Formula 1)}$$

Patent Document 1 discloses a method for methanation of a gas containing CO and $H_2$, which is characterized in that a methanation reactor in which a Cu-Zn-based low-temperature shift catalyst is arranged on an upstream side and a methanation catalyst is arranged on a downstream side is used when a gas containing CO and $H_2$ is converted into methane. Since a CO shift reaction (Formula 2) progresses in the upstream low-temperature shift reactor, it is thought that most of the carbon monoxide contained in the raw material gas reacts with water vapor and is converted to carbon dioxide, and the methanation reaction of carbon dioxide progresses on the downstream methanation catalyst.

$$CO + H_2O \rightarrow CO_2 + H_2 \qquad \text{(Formula 2)}$$

The methanation reaction has long been used for the purpose of removing carbon monoxide and carbon dioxide from hydrogen for ammonia synthesis, and it is known that catalysts supporting Ni, Ru, and the like exhibit high activity (Non-Patent Documents 1 and 2).

The methanation reaction in which carbon dioxide and hydrogen are reacted to obtain methane is an industrially established technique (e.g., Non-Patent Document 3), but there is still a problem in obtaining a fuel gas of a quality that can be used as a raw material for city gas.

Natural gas is commonly used as the raw material for city gas, and natural gas contains methane as a main component, as well as small amounts of ethane, propane, and butane. Natural gas does not normally contain hydrogen, and carbon dioxide is removed during the refining process of natural gas. In particular, in the case of city gas produced using liquefied natural gas as a raw material, hydrogen and carbon dioxide are almost completely removed in the process of liquefaction refining, and therefore they are substantially not contained in city gas.

Hydrogen and carbon dioxide being contained in city gas may cause the following problems.

Carbon dioxide is not only non-flammable, but also works to suppress combustion. Accordingly, if it is mixed in the fuel gas at a high concentration, it not only reduces the efficiency of gas transportation in gas pipeline due to the decrease in the heating value of the fuel gas, but also may cause a decrease in the efficiency of combustion equipment.

Although hydrogen is a fuel gas, its heating value per unit volume is only about one-third that of methane, which is the main component of city gas. Accordingly, when hydrogen is mixed in the fuel gas, which is mainly composed of methane, the heating value per unit volume decreases. Furthermore, hydrogen is known to have a large impact on combustion equipment because of its high burning velocity.

The methanation reaction of carbon dioxide (Formula 1) is an equilibrium reaction, and under normal industrial operating conditions, carbon dioxide and hydrogen cannot be completely converted to methane. The equilibrium conversion rate of carbon dioxide to methane when reacting a mixed gas with a stoichiometric ratio (hydrogen : carbon dioxide = 4:1) at normal pressure (0.1 MPa) is 95.0% at a reaction temperature of 300°C, 97.5% at a reaction temperature of 250°C, and 98.9% at a reaction temperature of 200°C.

Thus, under normal pressure, only a fuel gas containing a large amount of hydrogen can be obtained. Since the methanation reaction is an exothermic reaction, the lower the temperature is, the higher the equilibrium conversion rate is, but in the case of a catalytic reaction, the lower the temperature is, the lower the catalytic activity is. For this reason, there is a lower limit to the reaction temperature, and in the case of a normal methanation catalyst, a temperature of 250°C or higher is required in order to obtain a practical reaction rate (Non-Patent Document 4, Patent Documents 2 and 3).

Since the methanation reaction of carbon dioxide (Formula 1) is a reaction in which the amount of substance (number of moles) decreases, the higher the pressure is, the higher the equilibrium conversion rate is. When the methanation reaction is carried out at a high pressure, a fuel gas with high methane purity can be obtained, but there are problems such as a reaction facility that withstands high pressures being expensive, as well as a large amount of compression power for the raw material gas being required. If a methanation reaction is carried out using a catalyst with excellent low-temperature activity, it is possible to obtain a fuel gas with high methane purity without increasing the reaction pressure to an extreme degree, which is economically advantageous.

Patent Document 4 discloses a catalyst for hydrogen reduction of carbon dioxide in which nanoparticles are dispersed and supported in a powdery carrier, 90% or more of the nanoparticles being particles with a particle diameter of less than 10 nm, and the nanoparticles being at least one type of metal particle selected from the group consisting of Fe, Co, Ni, Cu, Ru, Rh, Pd, Ag, Ir, Pt, and Au, or material particles containing the metal particles.

This document discloses that sputtering is performed while mixing, rotating, or causing pendulum motion of a powdery carrier in a vacuum container having a polygonal internal cross section by rotating or causing pendulum motion of the vacuum container about a rotation axis substantially perpendicular to the cross section, whereby the nanoparticles can be dispersed and supported on the surface of the powdery carrier, and in a methanation catalyst prepared in this manner, a 100% $CO_2$ conversion rate is obtained at a reaction temperature of 200°C, and the methanation catalyst has more excellent low-temperature activity than a methanation catalyst obtained through a general impregnation method.

Patent Document 5 discloses a catalyst for hydrogen reduction of carbon dioxide, in which catalyst metal nanoparticles and metal oxides for suppressing particle growth of the catalyst metal nanoparticles are dispersed and supported on a carrier.

This document discloses that nanoparticles containing a metal and a metal oxide can be dispersed and supported on the surface of a carrier by using a target containing the metal and the metal oxide and performing sputtering while rolling the carrier, and this document discloses that in a methanation catalyst prepared in this manner, the metal nanoparticles have a smaller particle diameter and methanation activity is higher compared to a catalyst without metal oxides.

However, the supporting of the active metal using sputtering shown in these documents is easy to apply when the carrier is in the form of powder, but there is a problem in that the active metal can be supported only on the outermost surface of the carrier when using a carrier molded into a predetermined shape in advance.

Patent Document 6 discloses a methanation catalyst containing a carrier made of at least one type of metal oxide selected from the group consisting of titania, zirconia, and alumina, ceria particles supported on the carrier, and ruthenium particles supported on the carrier, in which the ceria particles have an average particle diameter of 8 nm or less, the amount of ceria particles supported is 0.3 to 10 parts by mass with respect to 100 parts by mass of the carrier, the ruthenium particles have an average particle diameter of 8 nm or less, and the amount of ruthenium particles supported is 0.5 to 5 parts by mass with respect to 100 parts by mass of the carrier.

According to this document, high catalytic activity is obtained when the coverage of the carrier surface by ceria particles and ruthenium particles is 1 to 80%, preferably 3 to 75%, and more preferably 5 to 70%.

However, none of Patent Documents 4 to 6 describes a method for producing a molded methanation catalyst having sufficient strength for practical use.

As a catalyst molding method, there are methods such as rolling granulation, tablet molding, and extrusion molding, but in any molding method, it is necessary to perform heat treatment after molding in order to impart sufficient strength, and therefore there is a risk that the catalytic activity will decrease in this process.

Activated alumina is widely used as a carrier for industrial catalysts, and is used as a carrier for methanation catalysts as well, because it is easy to obtain a molded body with high strength through a method such as performing rolling granulation on boehmite and thereafter firing the resulting granules in air to obtain activated alumina.

However, it is known that activated alumina changes its crystal structure at high temperatures of 1000°C or higher or under conditions of high partial water vapor pressure even at lower temperatures, resulting in a decrease in specific surface area, and the strength of the molded body decreases significantly accompanying this.

Patent Document 7 discloses that a catalyst in which ruthenium is supported on an activated alumina carrier containing 0.5 to 10 wt% of silica maintains stable catalytic activity and sufficient strength over a long period of time under conditions of a water vapor reforming reaction of hydrocarbons, and that a rapid decrease in activity and a significant decrease in strength are observed in a short period of time when activated alumina containing no silica is used as a carrier under the same conditions.

Patent Document 8 discloses a method for producing activated alumina with excellent heat resistance, in which an activated alumina powder, a molded body thereof, or a molded body containing activated alumina is caused to support an organic silicon compound, and then the supported organic silicon compound is oxidized or thermally decomposed.

Patent Document 9 discloses a method for producing activated alumina with high heat resistance, including:

a decomposition deposition step of bringing cyclic siloxane into contact with activated alumina in an oxidizing atmosphere of 100°C or more and 300° C or less to decompose and deposit the cyclic siloxane on the activated alumina; and a firing step of firing the activated alumina in an oxidizing atmosphere to form a silica coating on the activated alumina.

Activated alumina coated with silica has excellent heat resistance, but when used as a catalyst carrier, there is a problem in that the degree of dispersion of the supported metal decreases. Silica has a weaker interaction with the supported metal than activated alumina does, and with silica, the supported metal tends to coarsen.

Patent Document 10 discloses a composition containing zirconium oxide on a carrier based on alumina or aluminum oxyhydroxide, in which after firing at 900°C for 4 hours, zirconium oxide is in the form of particles attached on the carrier, and the particle diameter thereof is at most 10 nm.

It is disclosed that this composition is obtained, for example, by mixing a colloidal dispersion of a zirconium compound with alumina or aluminum oxyhydroxide, and then performing drying and firing, and a decrease in surface area after firing at 1000°C for 4 hours is less than that of alumina supporting zirconium oxide prepared through a known impregnation method.

However, this document does not disclose the effect that ruthenium being supported has on the ruthenium dispersion degree and the influence on the activity of a methanation catalyst, nor does it describe whether or not the phase change of alumina can be suppressed. Also, there is no specific description of a method for obtaining a molded body having industrially sufficient strength.

Patent Document 11 discloses an impregnating solution for producing a ruthenium catalyst, which is an aqueous solution containing a ruthenium compound and a compound of a group IVa element of the periodic table and has a pH of 3 or less, and a method for producing a ruthenium catalyst in which this impregnating solution is brought into contact with a carrier, a ruthenium component and a group IVa element component of the periodic table are supported on the carrier, and the resulting ruthenium-supported composition is dried and then fired.

However, this document only shows the particle diameter of the supported ruthenium as a result of electron microscopy, and although this document illustrates various reactions, such as a catalyst for selective hydrogenation of unsaturated compounds such as carbonyl compounds, aromatic compounds, olefins and dienes, an ammonia synthesis catalyst, an FT synthesis catalyst, CO and $CO_2$ methanation catalysts, catalysts for hydrogenation of CO and $CO_2$ to alcohols or the like, a nitro compound hydrogenation catalyst, a hydrocarbon hydrocracking catalyst, a catalyst for selective hydrogenation of aromatic amines, a NOx reduction purification catalyst, a water vapor reforming catalyst for hydrocarbons or the like, a low-temperature complete oxidation catalyst, a photosemiconductor catalyst, and an electrode catalyst, no specific catalytic activity is shown for any reaction.

Moreover, Patent Document 11 does not describe the crystal phase of zirconia supported on alumina and the effect this has on the phase change of alumina.

This document shows that, compared with the ruthenium particle diameter of a catalyst obtained by supporting zirconia on activated alumina and then supporting ruthenium, the ruthenium particle diameter of a catalyst obtained by impregnating activated alumina with an impregnating solution that contains a ruthenium compound and a zirconium compound and has a pH of 3 or less, and performing drying and firing is smaller, and it is explained that the reason for this is that ruthenium and zirconium form a complex-like compound in the impregnation solution.

However, in this method, the distributions of ruthenium and zirconium in the molded catalyst must be the same, and the distributions of ruthenium and zirconium cannot be controlled separately. In fact, Patent Document 11 describes that ruthenium is evenly and uniformly supported.

From the viewpoint of suppressing the phase change of alumina, zirconia needs to be supported in a sufficient concentration up to the central portion of the molded catalyst. On the other hand, it is preferable that a large amount of ruthenium, which serves as an active site of the catalyst, is supported near the surface of the molded catalyst. However, with the method described in Patent Document 11, it is difficult to separately control the distributions of ruthenium and zirconium. Patent Document 11 does not give any suggestion as to the significance of separately controlling the distributions of ruthenium and zirconium, or how to realize this.

As described above, regarding the methanation catalyst formed by supporting ruthenium as an active metal, it is a fact that a molded catalyst having high activity at low temperatures, sufficient strength for industrial use, and heat resistance under high temperature and high water vapor pressure conditions has not yet been established.

Prior Art Documents

Patent Documents

Patent Document 1: JP S60-235893A
Patent Document 2: JP 2015-124217A

Patent Document 3: JP 2018-135283A
Patent Document 4: JP 2009-131835A
Patent Document 5: JP 2019-48249A
Patent Document 6: JP 2019-76862A
Patent Document 7: JP S57-4232A
Patent Document 8: JP S50-24200A
Patent Document 9: JP 2020-132514A
Patent Document 10: JP 2011-513055A
Patent Document 11: JP H7-116516A

Non-Patent Documents

Non-Patent Document 1: Edited by the Society of Chemical Engineers, Chemical Process Collection, 1970, p. 153
Non-Patent Document 2: Catalysis Society of Japan, Catalysis Handbook, 2008, p. 535
Non-Patent Document 3: Kawagoe, Matsuda, Matsushima, and Uematsu, Hitachi Hyoron, Vol. 68, No. 10, 1986, p. 73
Non-Patent Document 4: E.I. Koytsoumpa and S. Karellas, Renewable and Sustainable Energy Reviews, Vol. 94, 2018, p. 536

Disclosure of the Invention

Problem to be Solved by the Invention

In view of the above problems, the problem to be solved by the present invention is to provide a molded catalyst serving as a methanation catalyst that supports ruthenium as an active metal and has high activity at low temperatures, sufficient strength for industrial use, and heat resistance under high temperature and high water vapor pressure conditions, and a method for producing the same.

Means for Solving Problem

A characteristic configuration of a methanation catalyst molded body according to the present invention includes: an activated alumina molded body; and zirconia and ruthenium supported on the activated alumina molded body, in which the amount of zirconia supported is 3 to 10 parts by mass with respect to 100 parts by mass of the activated alumina molded body, the amount of ruthenium supported is 0.1 to 5 parts by mass with respect to 100 parts by mass of the activated alumina molded body, and the carbon dioxide methanation catalyst molded body is a molded body having a particle diameter of 2 to 20 mm.

According to this characteristic configuration, the methanation catalyst molded body (hereinafter referred to as simply a catalyst molded body in some cases) has high activity for a methanation reaction at low temperatures, sufficient strength for industrial use, and heat resistance under high temperature and high water vapor pressure conditions.

In the above-described methanation catalyst molded body, when ruthenium is supported on the activated alumina molded body in an eggshell shape having a shell portion with a thickness of 0.2 to 0.4 mm, the amount of zirconia supported in a central portion is 50% or more and less than 100% using the average amount of zirconia supported in the entire activated alumina molded body as a reference, and the amount of zirconia supported is more than 100% in the shell portion where ruthenium is supported, the methanation catalyst molded body has particularly high activity for a methanation reaction at low temperatures, sufficient strength for industrial use, and heat resistance under high temperature and high water vapor pressure conditions.

Also, when the zirconia is present mainly as a tetragonal crystal in the above-described methanation catalyst molded body, the methanation catalyst molded body has particularly high activity for a methanation reaction at low temperatures, sufficient strength for industrial use, and heat resistance under high temperature and high water vapor pressure conditions.

Furthermore, in the methanation catalyst molded body, it is preferable that the content of cerium oxide is 5 parts by mass or less with respect to 100 parts by mass of the activated alumina molded body, because the methanation catalyst molded body will have high activity for a methanation reaction at low temperatures. Also, in the above-described methanation catalyst molded body, it is preferable that a degree of phase transformation to alpha-type, which is measured through a procedure of firing the activated alumina molded body supporting zirconia at a temperature of 1050°C for 6 hours in air, and then measuring the degree of phase transformation to alpha-type through X-ray diffraction measurement using Cu-Ka rays as a radiation source, is 10% or less, or the degree of phase transformation to alpha-type, which is measured through a procedure of firing the methanation catalyst molded body at a temperature of 1050°C for 6 hours in air, and then measuring the degree of phase transformation to alpha-type through X-ray diffraction measurement using Cu-Ka rays as a radiation source, is 10% or less,-because the methanation catalyst molded body has strength that is sufficient for industrial use, and the heat resistance under high temperature and high water vapor pressure conditions particularly improves. That is, in the above-described methanation catalyst molded body, when the degree of phase transformation to alpha-type, which is measured through the procedure of firing the activated alumina molded body, in which the content of cerium oxide is 5 parts by mass or less with respect to 100 parts by mass of the activated alumina molded body and zirconia is supported, at 1050°C for 6 hours in air, and then measuring the degree of phase transformation to alpha-type through X-ray diffraction measurement using Cu-Ka rays as a radiation source, is 10% or less, or when the degree of phase transformation to alpha-type, which is measured through the procedure of firing the methanation catalyst molded body at 1050°C for 6 hours in air, and then measuring the degree of phase transformation to alpha-type through X-ray diffraction measurement using Cu-Ka rays as a radiation source, is 10% or less, the methanation catalyst molded body has high activity for a methanation reaction at low temperatures and sufficient strength for industrial use, and heat resistance under high temperature and high water vapor pressure conditions is particularly improved.

A characteristic configuration of a method for producing a methanation catalyst molded body of the present invention includes: a zirconium impregnation step of impregnating an activated alumina molded body having a particle diameter of 2 to 20 mm with an aqueous solution in which a water-soluble compound of zirconium is dissolved, to obtain a zirconium impregnated body; a drying step of drying the zirconium-impregnated body to obtain a dry body; a firing step of firing the dry body at 500 to 800°C in air to obtain activated alumina in which zirconia is supported in a dispersed manner; a ruthenium impregnation step of impregnating the activated alumina in which the zirconia is supported in a dispersed manner with an aqueous solution in which a water-soluble compound of ruthenium is dissolved, to obtain a ruthenium impregnated body; and a ruthenium immobilization step of immobilizing the ruthenium by drying the ruthenium impregnated body.

According to this method, it is possible to, with an economically advantageous method, produce a carbon dioxide methanation catalyst molded body having high activity for a methanation reaction at low temperatures, sufficient strength for industrial use, and heat resistance under high temperature and high water vapor pressure conditions.

In the above method for producing a methanation catalyst molded body, when the zirconium impregnation step is performed using an aqueous solution in which a water-soluble compound of zirconium is dissolved and that is acidified with nitric acid, it is possible to produce a methanation catalyst molded body that is excellent in low-temperature activity for a methanation reaction, strength sufficient for industrial use, and heat resistance under high temperature and high water vapor pressure conditions.

Brief Description of the Drawings

FIG. 1 shows an electron probe microanalysis (EPMA) measurement result showing distributions of Al, Zr, and Ru in a cross section of a methanation catalyst molded body according to an example of the present invention.
FIG. 2 shows an electron probe microanalysis (EPMA) measurement result showing distributions of Al, Zr, and Ru in a cross section of a methanation catalyst molded body according to an example of the present invention.
FIG. 3 is a diagram showing an X-ray diffraction pattern of a catalyst according to an example and a comparative example of the present invention.

Best Mode for Carrying Out the Invention

Hereinafter, embodiments of the methanation catalyst molded body and the method for producing the methanation catalyst molded body according to the present invention will be described.

The main component of the methanation catalyst molded body of the present invention is activated alumina, which is transition alumina represented by a γ-type and an η-type. Activated alumina undergoes a phase transformation to an α-type through a method such as firing at a high temperature of 1000°C or higher, but since α-type alumina has a small specific surface area, zirconia and ruthenium cannot be supported in a highly dispersed manner, and therefore α-type alumina is not suitable as a carrier for the methanation catalyst molded body of the present invention. In the methanation catalyst molded body of the present invention, it is preferable that the alumina does not contain α-type alumina, or that even if the alumina contains α-type alumina, the mass ratio thereof with respect to the total alumina is 5% or less.

The methanation catalyst molded body of the present invention contains zirconia and ruthenium supported on the activated alumina molded body, and the amount of zirconia supported is 3 to 10 parts by mass with respect to 100 parts by mass of the activated alumina molded body, and the amount of ruthenium supported is 0.1 to 5 parts by mass with respect to 100 parts by mass of the activated alumina molded body.

If the amount of zirconia supported is less than 3 parts by mass with respect to 100 parts by mass of the activated alumina molded body, there is a risk that the effect of stabilizing the activated alumina will decrease, and the activated alumina will undergo a phase transformation to the $\alpha$-type under high temperature and high partial water vapor pressure conditions, which are the reaction conditions for the methanation catalyst molded body, and problems may occur, such as a decrease in catalytic activity and the catalyst turning into powder.

If the amount of zirconia supported is greater than 10 parts by mass with respect to 100 parts by mass of the activated alumina molded body, there is a risk that pores formed in the activated alumina molded body will be clogged with zirconia, resulting in a decrease in gas diffusibility, whereby catalytic activity may decrease.

The crystal phases of zirconia include tetragonal crystals, monoclinic crystals, and cubic crystals, and at 1100°C or lower, the monoclinic crystals are stable. However, in the methanation catalyst molded body of the present invention, zirconia is supported in a highly dispersed state mainly in the form of tetragonal crystals. Monoclinic zirconia may be contained in the methanation catalyst molded body of the present invention, but when zirconia is present mainly as monoclinic crystals, the degree of dispersion of zirconia supported on alumina is low, and therefore the effect of stabilizing the activated alumina is not sufficiently obtained in some cases.

If the amount of ruthenium supported is less than 0.1 parts by mass with respect to 100 parts by mass of the activated alumina molded body, sufficient methanation activity cannot be obtained. On the other hand, if the amount of ruthenium supported is more than 5 parts by mass with respect to 100 parts by mass of the activated alumina molded body, the degree of dispersion of the supported ruthenium becomes low, and the methanation activity corresponding to the supported amount cannot be obtained. Also, the amount of ruthenium supported is preferably 0.5 to 2 parts by mass with respect to 100 parts by mass of the activated alumina molded body from the viewpoint of obtaining sufficient methanation activity that is more consistent with the amount of ruthenium supported.

The methanation catalyst molded body of the present invention is a molded body having a particle diameter of 2 to 20 mm. Here, a particle diameter of 2 to 20 mm means that if the molded body is spherical, the diameter thereof is in the range of 2 to 20 mm, if the molded body is cylindrical, the diameter and the length are in the range of 2 to 20 mm, and if the molded body has another shape, the hydrodynamic equivalent diameter is in the range of 2 to 20 mm.

If the particle diameter of the methanation catalyst molded body is smaller than 2 mm, the pressure loss increases when the reaction gas flows through a reaction tank filled with the methanation catalyst molded body, and the economic efficiency of the methanation process deteriorates. On the other hand, when the particle diameter is larger than 20 mm, the methanation activity decreases because the geometrical surface area of the molded body becomes relatively small.

It is preferable that ruthenium, which is responsible for catalytic activity, is supported at a higher concentration in the surface portion than in the center portion of the catalyst molded body. This is because ruthenium in the vicinity of the surface of the catalyst molded body acts more effectively as a catalyst than ruthenium in the center portion of the catalyst molded body due to the problem of diffusion of reaction gas in the catalyst molded body. However, when consideration is given to the fact that the outermost surface of the catalyst molded body is likely to wear due to friction, attrition of ruthenium due to wear increases if ruthenium is supported only on the outermost surface, and a certain dispersion degree is ensured, ruthenium is evenly supported up to a certain depth from the surface of the catalyst molded body, and if it is not supported toward the center or the supported concentration is lowered, high methanation activity is easy to obtain with a small amount of ruthenium supported.

More specifically, in the catalyst molded body, when ruthenium is supported on the activated alumina molded body in an eggshell shape having a shell portion with a thickness of 0.2 to 0.4 mm, or in other words, when ruthenium is supported on the shell portion 0.2 to 0.4 mm from the surface of the catalyst molded body, high methanation activity is easily obtained with a small amount of ruthenium supported.

On the other hand, zirconia has the effect of stabilizing activated alumina, in addition to the effect of increasing the degree of dispersion of ruthenium, and therefore zirconia needs to be supported up to the center portion of the catalyst molded body.

More specifically, in the catalyst molded body, when the amount of zirconia supported in the central portion is 50% or more and less than 100% using the average amount of zirconia supported in the entire activated alumina molded body as a reference, it is easy to stabilize the activated alumina, and when the amount of zirconia supported is higher than 100% in the shell portion where ruthenium is supported, the dispersion degree of ruthenium increases, and high methanation activity is easily obtained.

The method for producing a methanation catalyst molded body of the present invention includes a zirconium impregnation step of impregnating an activated alumina molded body having a particle diameter of 2 to 20 mm with an aqueous solution in which a water-soluble compound of zirconium is dissolved, to obtain a zirconium impregnated body; a drying step of drying the zirconium impregnated body to obtain a dry body; a firing step of firing the dry body at 500 to 800°C in air to obtain activated alumina in which zirconia is supported in a dispersed manner; a ruthenium impregnation step of impregnating the activated alumina in which the zirconia is supported in a dispersed manner with an aqueous solution in which a water-soluble compound of ruthenium is dissolved, to obtain a ruthenium impregnated body; and a ruthenium immobilization step of immobilizing ruthenium by drying the ruthenium impregnated body.

The activated alumina molded body is transitional alumina represented by the $\gamma$-type and the $\eta$-type, and is molded into a spherical or cylindrical shape with a diameter of 2 mm to 20 mm. Such a molded body is obtained through a rolling granulation method or a tablet molding method.

Zirconium nitrate ($Zr(NO_3)_4$), zirconium nitrate oxide ($Zr(NO_3)_2O$), zirconium acetate ($Zr(CH_3COO)_4$), zirconium acetate oxide ($Zr(CH_3COO)_2O$), and the like can be used as water-soluble compounds of zirconium.

Some of the water-soluble compounds of zirconium are not sufficiently soluble in water, and some aqueous solutions of the water-soluble compounds of zirconium are not sufficiently stable. In such a case, nitric acid, hydrochloric acid, or the like may be added to the aqueous solution. An aqueous solution acidified with nitric acid is particularly preferable because the water-soluble compound of zirconium is stabilized and zirconia is easily supported in the methanation catalyst molded body with a suitable distribution.

Although the temperature and time of the zirconium impregnation step are not particularly limited, the zirconium impregnation step can be performed, for example, at room temperature for about 1 to 20 hours.

Although the temperature and time of the drying step are not particularly limited, the drying step can be performed, for example, at 80°C to 200°C for about 1 to 20 hours.

If the temperature in the firing step is too low, there is a risk that the zirconium compound will not decompose sufficiently and will be eluted in the ruthenium supporting step, and even if the temperature in the firing step is too high, there is a risk that sintering of the activated alumina will proceed and the specific surface area thereof will decrease. Accordingly, the temperature is preferably 500°C or more and 800°C or less.

If the time for the firing step is too short, there is a risk that the zirconium compound will not sufficiently decompose, and if the time for the firing step is too long, it will be economically disadvantageous, and there is a risk that the specific surface area of the activated alumina will decrease, and therefore the time is preferably 1 hour or more and 20 hours or less.

Air may be used as the gas flowing in the firing step, but oxygen or nitrogen may also be added as necessary to adjust the oxygen concentration.

Ruthenium chloride ($RuCl_3$), ruthenium nitrate ($Ru(NO_3)_3$), and the like can be used as water-soluble compounds of ruthenium.

The temperature and time of the ruthenium impregnation step are not particularly limited, and for example, the ruthenium impregnation step can be performed at room temperature for about 1 to 20 hours.

The ruthenium immobilization step of immobilizing ruthenium can be performed using any method as long as the impregnated ruthenium can be fixed on the molded body without flowing out and does not leave a residue that inhibits activity on the catalyst. However, for example, the ruthenium immobilization step can be implemented by immersing the ruthenium-impregnated body in an alkaline solution of sodium hydroxide or the like to fix the ruthenium as a hydroxide, further performing reduction using a reducing agent such as hydrazine to form metallic ruthenium, washing the metallic ruthenium to remove sodium ions, chloride ions, nitrate ions, and the like, and then drying the washed metallic ruthenium in air at about 60°C to 100°C.

The methanation catalyst molded body of the present invention has high activity for methanation of carbon dioxide. The reaction of hydrogen and carbon dioxide to obtain methane is accompanied by a relatively large amount of heat generation, and therefore if the reaction is carried out adiabatically, the temperature of the catalyst layer may rise by approximately 200°C to 400°C. When the temperature of the catalyst layer rises, the supported ruthenium clumps together, resulting in a decrease in catalytic activity, and activated alumina undergoing sintering and phase change, which may reduce the strength of the catalyst.

For this reason, when the methanation reaction of hydrogen and carbon dioxide is carried out, part of the reactor outlet gas is recycled to the reactor inlet to mitigate heat generation. In this case, the gas introduced into the methanation catalyst molded body contains hydrogen and carbon dioxide, as well as methane, water vapor, and carbon monoxide produced through the reverse reaction of the CO shift reaction. However, since the catalyst of the present invention exhibits high methanation activity even in the presence of water vapor, and exhibits activity for methanation of carbon monoxide as well, the catalyst of the present invention can be suitably used even under the condition of a methanation reaction with recycling.

In the methanation reaction using the methanation catalyst molded body of the present invention, there are no particular restrictions on the conditions of use as long as the catalyst exhibits activity, but the methanation reaction is normally carried out at a temperature of 200°C to 600°C and under a pressure of normal pressure to 10 MPa.

Examples

Hereinafter, the present invention will be described in more detail based on examples and comparative examples, but the present invention is not limited to the following examples.

Example 1

40 g of activated alumina (manufactured by Kishida Chemical Co., Ltd., catalyst aluminum oxide (activated type), 4 to 6 mm spherical molded body) was immersed in 40 g of an aqueous solution of zirconium acetate oxide (manufactured by Tokyo Kasei Kogyo, containing 20 mass% in terms of zirconium oxide), and was impregnated for 15 hours to obtain a zirconium-impregnated body. This zirconium -impregnated body was evaporated to dryness on a hot plate and then dried in a dryer maintained at 125°C for 1 hour to obtain a dry body. This dry body was loaded into an electric furnace, heated from normal temperature to 700°C over 3 hours with a flow of air, and was kept at 700°C for 4 hours and fired. Thereafter, it was allowed to cool to normal temperature over 3 hours to obtain a zirconia-supported alumina A.

98 parts by mass of the zirconia-supported alumina A was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst A.

Example 2

3.25 g of zirconium nitrate oxide dihydrate ($Zr(NO_3)_2O \cdot 2H_2O$) was dissolved in dilute nitric acid prepared by mixing 2.2 g of 60% nitric acid and 20 g of pure water to obtain an aqueous solution in which a zirconium compound is dissolved. 30 g of the same activated alumina as used in Example 1 was immersed in the above aqueous solution and impregnated for 15 hours to obtain a zirconium-impregnated body. This zirconium-impregnated body was evaporated to dryness on a hot plate and then dried in a dryer maintained at 125°C for 1 hour to obtain a dry body. This dry body was loaded into an electric furnace, heated from normal temperature to 700°C over 3 hours with a flow of air, and was kept at 700°C for 4 hours and fired. Thereafter, it was allowed to cool to normal temperature over 3 hours to obtain a zirconia-supported alumina B.

98 parts by mass of the zirconia-supported alumina B was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction using a 0.3% aqueous hydrazine solution, and washed with hot water at 80°C, and then was dried at 80°C for 4 hours to obtain a catalyst B.

Example 3

6.51 g of zirconium nitrate oxide dihydrate was dissolved in dilute nitric acid prepared by mixing 6.4 g of 60% nitric acid and 18 g of pure water, to obtain an aqueous solution in which a zirconium compound is dissolved. 30 g of the same activated alumina as used in Example 1 was immersed in the above aqueous solution and impregnated for 15 hours to obtain a zirconium-impregnated body. This zirconium -impregnated body was evaporated to dryness on a hot plate and then dried in a dryer maintained at 125°C for 1 hour to obtain a dry body. This dry body was loaded into an electric furnace, heated from normal temperature to 700°C over 3 hours with a flow of air, and was kept at 700°C for 4 hours and fired. Thereafter, it was allowed to cool to normal temperature over 3 hours, to obtain a zirconia-supported alumina C.

98 parts by mass of the zirconia-supported alumina C was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst C.

Comparative Example 1

A borosilicate glass petri dish with an outer diameter of 60 mm and a height (outer dimension) of 14 mm was placed in the center of a borosilicate glass petri dish with an inner diameter of 138 mm and a height (inner dimension) of 22 mm. 1.6 g of decamethylcyclopentasiloxane was dripped onto the inner petri dish, 40 g of the same activated alumina as in Example 1 was added evenly to the outer petri dish, and the outer petri dish was covered with a lid.

This petri dish was placed in an electric furnace, heated from normal temperature to 200°C over 1.5 hours, kept at 200°C for 1 hour, and allowed to cool to normal temperature over about 1 hour. Note that in this process, air flowed in the electric furnace at a flow rate of 1 liter per minute.

After being allowed to cool, the petri dish was removed from the electric furnace, the activated alumina was transferred to an alumina firing container, the temperature was raised from normal temperature to 500°C over 1.5 hours, and firing was performed at 500°C for 1 hour to obtain a silica-coated alumina D.

98 parts by mass of the silica-coated alumina D was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst D.

Comparative Example 2

98 parts by mass of the same activated alumina as used in Example 1 was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst E.

Heat Resistance Evaluation Result

The zirconia-supported aluminas A, B, and C, the silica-coated alumina D, and untreated activated alumina (referred to as alumina E) were each subjected to high-temperature firing in air at 1050°C for 6 hours. Table 1 shows the $ZrO_2$ or $SiO_2$ content, BET specific surface area (before and after high-temperature firing), and degree of phase transformation to alpha-type of each sample.

[Table 1]

| Sample | $ZrO_2$ (wt%) | $SiO_2$ (wt%) | BET specific surface area $(m^2/g)$ | Degree of transformation to $\alpha$ (%) |
|---|---|---|---|---|
| Zirconia-supported alumina A | 8.2 | - | $144 \rightarrow 64.4$ | 0 |
| Zirconia-supported alumina B | 3.5 | - | $150 \rightarrow 56.3$ | 1.5 |
| Zirconia-supported alumina C | 7.3 | - | $144 \rightarrow 61.3$ | 0 |
| Silica-coated alumina D | - | 2.8 | $163 \rightarrow 78.6$ | 21 |
| Alumina E | - | - | $162 \rightarrow 27.2$ | 59 |
| (Note) The BET specific surface area is written in the format before high-temperature firing $\rightarrow$ after high-temperature firing. Also, the degree of phase transformation to alpha-type is the value after high-temperature firing. | | | | |

Method for Measuring $SiO_2$ Content and $ZrO_2$ Content

Each sample of the zirconia-supported aluminas A, B, and C, and the silica-coated alumina D was subjected to acid decomposition, Si and Zr were quantified through ICP emission spectrometry, and the contents were determined by converting Si and Zr into oxides.

Method for Measuring BET Specific Surface Area

For each sample before and after high-temperature firing, the BET specific surface area was measured through a BET one-point method using a nitrogen adsorption amount under the condition of relative pressure $(P/P_0) = 0.3$ at liquid nitrogen temperature.

Method for Measuring Degree of phase transformation to alpha-type

For each sample after high-temperature firing, X-ray diffraction measurement was performed, and regarding the (012) diffraction line (25.6°) of $\alpha$-alumina, the degree of phase transformation to alpha-type was calculated as the ratio of the diffraction line intensity of each sample to the diffraction line intensity of pure $\alpha$-alumina. Note that the X-ray diffraction measurement was performed under the following conditions using an X-ray diffractometer (XRD-6100 manufactured by Shimadzu Corporation) equipped with a graphite monochromator.

X-ray source: Cu-Ka rays (0.1542 nm) emitted from an X-ray tube (Cu target, tube voltage 40 kV, tube current 40 mA).
Measurement conditions: Step scan method, 0.02° steps, cumulative time at each step: 1.2 seconds, detection slit:

0.15 mm.

Evaluation Results of Ru Dispersion Degree and Crushing Strength

The metal dispersion degree of the supported ruthenium and the crushing strength of each of the catalysts A, B, C, D, and E were evaluated.

Table 2 shows the Ru, $ZrO_2$, $SiO_2$, and $Al_2O_3$ contents, BET specific surface area, ruthenium dispersion degree, and crushing strength of each catalyst.

[Table 2]

| Sample | Ru (wt%) | $ZrO_2$ (wt%) | $SiO_2$ (wt%) | $Al_2O_3$ (wt%) | Ru dispersion degree (-) | Crushing strength (N) |
|---|---|---|---|---|---|---|
| Catalyst A | 1.63 | 8.1 | - | 90.3 | 0.69 | 233 |
| Catalyst B | 1.64 | 3.3 | - | 95.1 | 0.61 | 205 |
| Catalyst C | 1.61 | 7.0 | - | 91.4 | 0.73 | 222 |
| Catalyst D | 1.64 | - | 3.0 | 95.3 | 0.42 | 197 |
| Catalyst E | 1.66 | - | - | 98.3 | 0.64 | 203 |

Ru, $ZrO_2$, $SiO_2$, and $Al_2O_3$ Content Measurement Method

Each sample after supporting ruthenium was subjected to acid decomposition, the Ru, Si, and Zr of each sample were quantified through ICP emission spectrometry, and the contents were obtained by using Ru as-is and converting Si and Zr into oxides.

Method for Measuring Surface Area of Metallic Ruthenium

For each sample after supporting ruthenium, the CO adsorption amount was measured according to a metal surface area measurement method performed through the CO pulse method (Catalysis Society of Japan Reference Catalyst Committee, "Catalyst", vol. 31, p. 317, 1989), and the CO adsorption amount was shown as the CO adsorption amount per metallic ruthenium (molar ratio of CO/Ru) (i.e., the ruthenium dispersion degree).

Method for Measuring Crushing Strength

Using a desktop load tester FTN1-13A manufactured by Aikoh Engineering Co., Ltd., the crushing strength of 15 molded catalyst particles was measured, and the average value thereof was used.

Evaluation Result of Methanation Activity

The methanation activity of each of the catalysts A, B, C, D and E was evaluated. Table 3 shows the results.

[Table 3]

| Sample | $CO_2$ conversion rate (%) | |
|---|---|---|
| | 225°C | 250°C |
| Catalyst A | 34.1 | 48.4 |
| Catalyst B | 35.6 | 48.1 |
| Catalyst C | 35.5 | 49.6 |
| Catalyst D | 10.9 | 19.9 |
| Catalyst E | 18.0 | 33.4 |

Method for Evaluating Methanation Activity

A stainless steel reaction tube (inner diameter: 24 mm) was filled with 5 mL of catalyst to form a catalyst layer. Then, while heating to maintain the temperature of this catalyst layer at 250°C, a reducing gas obtained by mixing nitrogen gas and 10% hydrogen gas (by volume) flowed therethrough at 150 liters per hour (volume in a standard state of 0°C and 1 atm, the same applies hereinafter), and the reduction treatment was performed for 3 hours.

After the reduction treatment, the temperature of the catalyst layer was changed to 225°C, the pressure inside the reaction tube was kept at 0.7 MPa (absolute pressure), and nitrogen gas (test gas) containing 2% carbon dioxide and 8% hydrogen (both by volume) flowed through the catalyst layer at a flow rate of 150 liters per hour, and the concentrations of carbon dioxide, hydrogen, nitrogen, and methane in the catalyst layer outlet gas were analyzed using a gas chromatograph (Shimadzu GC-14B, with a TCD detector). Thereafter, the temperature of the catalyst layer was changed to 250°C while the test gas flowed therethrough, and the catalyst layer outlet gas was similarly analyzed using the gas chromatograph. The conversion rate of $CO_2$ in the test gas was calculated using the following formula based on the methane and carbon dioxide concentrations (both vol%) in the catalyst layer outlet gas. Note that carbon monoxide was not detected in the catalyst layer outlet gas.

$$(CO_2 \text{ conversion rate } [\%]) = 100 \times (CH_4 \text{ concentration})/\{(CH_4 \text{ concentration})+(CO_2 \text{ concentration})\}$$

Evaluation of Examples and Comparative Examples

The results of the heat resistance evaluation will be considered next.

The BET specific surface area after high-temperature firing was 27.2 $m^2$/g for the alumina E and 78.6 $m^2$/g for the silica-coated alumina D, while the zirconia-supported aluminas A, B, and C had BET specific surface areas of 64.4 $m^2$/g, 56.3 $m^2$/g, and 61.3 $m^2$/g, respectively. In the zirconia-supported aluminas A, B, and C used in the examples, the decrease in BET specific surface area after high-temperature firing was significantly smaller than that of the alumina E, and the specific surface area was maintained at a level close to that of the silica-coated alumina D.

The degree of phase transformation to alpha-type after high-temperature firing was 59% for the alumina E and 21% for the silica-coated alumina D, whereas it was 1.5% for the zirconia-supported alumina B, and no $\alpha$-alumina peak was observed in the X-ray diffraction measurements for the zirconia-supported aluminas A and C. That is, the zirconia-supported aluminas A, B, and C exhibit better heat resistance than the alumina E as well as the silica-covered alumina D as far as the degree of phase transformation to alpha-type is concerned.

The above results were obtained by evaluating the heat resistance when ruthenium is not supported, but since sintering and phase-change of alumina progress regardless of whether or not ruthenium is included, the heat resistances when ruthenium is supported, that is, the heat resistances of the catalysts A, B, C, D, and E, are considered to be the same as above.

The concentration of ruthenium supported on the catalysts A, B, C, D, and E was 1.61 to 1.66 wt%, and was more or less the same when using any of the zirconia-supported aluminas A, B, and C, the silica-coated alumina D, and the alumina E. On the other hand, the dispersion degree of supported ruthenium was 0.64 when the alumina E was used, whereas it was significantly reduced to 0.42 when the silica-coated alumina D was used. It can be understood that when the surface of alumina is coated with silica, the heat resistance is improved, but the dispersion degree of the supported metal is significantly reduced.

The dispersion degrees of supported metals of the catalysts A, B, and C using zirconia-supported alumina of the present invention were 0.69, 0.61, and 0.73, respectively, which were equal to or higher than that of the catalyst E. In particular, the catalysts A and C, which had high zirconia contents, exhibited higher ruthenium dispersion degrees than the catalyst E.

The crushing strengths of the catalysts A, B, and C of the present invention are equal to or higher than that of the catalyst E, in which only ruthenium is supported on activated alumina, and an improvement in strength relative to the catalyst E was observed in the catalysts A and C, which have particularly high zirconia contents.

The methanation activity of the catalysts A, B, and C of the present invention was significantly higher than that of the catalyst E, in which only ruthenium was supported on activated alumina. On the other hand, the methanation activity of the catalyst D, in which ruthenium was supported on silica-coated alumina D, was clearly lower than that of the catalyst E. Comparative Example 3

4.04 g of cerium nitrate hexahydrate (Ce(NO$_3$)$_3$·6H$_2$O) was dissolved in 24 g of pure water to obtain an aqueous solution in which a cerium compound is dissolved. 32 g of the same activated alumina as used in Example 1 was immersed in the above aqueous solution and impregnated for 15 hours to obtain an impregnated body. After the impregnated body was evaporated to dryness on a hot plate, it was dried in a dryer maintained at 125°C for 1.5 hours to obtain a dry body. This dry body was loaded into an electric furnace, heated from normal temperature to 700°C over 3 hours with a flow of air, and was kept at 700°C for 4 hours and fired. Thereafter, it was allowed to cool to normal temperature over 3 hours to obtain a ceria-supported alumina F.

98 parts by mass of ceria-supported alumina F was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction using a 0.3% aqueous hydrazine solution, and washed with hot water at 80°C, and was dried for 4 hours at 80°C to obtain a catalyst F.

Example 4

3.47 g of zirconium nitrate oxide dihydrate and 4.04 g of cerium nitrate hexahydrate were dissolved in dilute nitric acid obtained by mixing together 3.2 g of 60% nitric acid and 24 g of pure water, to obtain an aqueous solution in which a zirconium compound and a cerium compound are dissolved. 32 g of the same activated alumina as used in Example 1 was immersed in the above aqueous solution and impregnated for 15 hours to obtain an impregnated body. After the impregnated body was evaporated to dryness on a hot plate, it was dried in a dryer maintained at 125°C for 1.5 hours to obtain a dry body. This dry body was loaded into an electric furnace, heated from normal temperature to 700°C over 3 hours with a flow of air, and was kept at 700°C for 4 hours and fired. Thereafter, it was allowed to cool to normal temperature over 3 hours to obtain a ceria-zirconia-supported alumina G.

98 parts by mass of the ceria-zirconia-supported alumina G was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst G.

Example 5

3.47 g of zirconium nitrate oxide dihydrate and 8.08 g of cerium nitrate hexahydrate were dissolved in dilute nitric acid obtained by mixing 3.2 g of 60% nitric acid and 24 g of pure water, to obtain an aqueous solution in which a zirconium compound and a cerium compound are dissolved. 32 g of the same activated alumina as used in Example 1 was immersed in the above aqueous solution and impregnated for 15 hours to obtain an impregnated body. After the impregnated body was evaporated to dryness on a hot plate, it was dried in a dryer maintained at 125°C for 1.5 hours to obtain a dry body. This dry body was loaded into an electric furnace, heated from normal temperature to 700°C over 3 hours with a flow of air, and was kept at 700°C for 4 hours and fired. Thereafter, it was allowed to cool to normal temperature over 3 hours, and a ceria-zirconia-supported alumina H was obtained.

98 parts by mass of ceria-zirconia-supported alumina H was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst H.

Example 6

13.08 g of zirconium dichloride oxide dihydrate ($ZrCl_2O \cdot 2H_2O$) was dissolved in 25 g of pure water to obtain an aqueous solution in which a zirconium compound is dissolved. 50 g of the same activated alumina as used in Example 1 was immersed in the above aqueous solution and impregnated for 15 hours to obtain an impregnated body. The impregnated body was evaporated to dryness on a hot plate and then dried in a dryer maintained at 120°C for 1 hour to obtain a dry body. This dry body was loaded into an electric furnace, heated from normal temperature to 500°C over 3 hours with a flow of air, and was kept at 500°C for 2 hours and fired. Thereafter, it was allowed to cool to normal temperature over 3 hours to obtain a zirconia-supported alumina I.

98 parts by mass of the zirconia-supported alumina I was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, washed with hot water at 80°C, and then was dried at 80°C for 4 hours to obtain a catalyst I.

Heat Resistance Evaluation Result

The ceria-supported alumina F, the ceria-zirconia-supported aluminas G and H, and the zirconia-supported alumina I were each subjected to high-temperature firing in air at 1050°C for 6 hours. Table 4 shows the $ZrO_2$ and $CeO_2$ contents, BET specific surface area (before and after high-temperature firing), and degree of phase transformation to alpha-type of each sample.

[Table 4]

| Sample | ZrO$_2$ (wt%) | CeO$_2$ (wt%) | BET specific surface area (m$^2$/g) | Degree of transformation to $\alpha$ (%) |
|---|---|---|---|---|
| Ceria-supported alumina F | - | 4.8 | 144 → 46.3 | 1.9 |
| Ceria-zirconia-supported alumina G | 3.5 | 4.2 | 140 → 52.9 | 0 |
| Ceria-zirconia-supported alumina H | 3.2 | 8.8 | 133 → 48.7 | 0 |
| Zirconia-supported alumina I | 5.2 | - | 151 → 47.3 | 8.4 |
| (Note) The BET specific surface area is written in the format before high-temperature firing → after high-temperature firing. Also, the degree of phase transformation to alpha-type is the value after high-temperature firing. | | | | |

Measuring Method

The ZrO$_2$ content and CeO$_2$ content of each sample of the ceria-supported alumina F, the ceria-zirconia-supported aluminas G and H, and the zirconia-supported alumina I were obtained by performing acid decomposition, quantifying Ce and Zr through ICP emission spectrometry, and converting Ce and Zr to oxides. The method for measuring the BET specific surface area and the degree of phase transformation to alpha-type is the same as in Table 1.

Evaluation Results of Ru Dispersion Degree and Crushing Strength

For each of the catalysts F, G, H, and I, the metal dispersion degree of the supported ruthenium and the crushing strength were evaluated.
Table 5 shows the Ru, CeO$_2$, ZrO$_2$, and Al$_2$O$_3$ contents, BET specific surface area, ruthenium dispersion degree, and crushing strength of each catalyst.

[Table 5]

| Sample | Ru (wt%) | ZrO$_2$ (wt%) | CeO$_2$ (wt%) | Al$_2$O$_3$ (wt%) | Ru dispersion degree (-) | Crushing strength (N) |
|---|---|---|---|---|---|---|
| Catalyst F | 1.83 | - | 4.5 | 93.7 | 0.46 | 149 |
| Catalyst G | 1.90 | 3.2 | 4.7 | 90.2 | 0.51 | 182 |
| Catalyst H | 1.75 | 2.9 | 8.9 | 86.5 | 0.57 | 231 |
| Catalyst I | 2.06 | 5.3 | - | 92.6 | 0.65 | 140 |

Measuring Method

The Ru, SiO$_2$, ZrO$_2$, and Al$_2$O$_3$ contents of each of the catalysts F, G, H, and I were obtained by performing acid decomposition, quantifying Ru, Ce, Zr, and Al through ICP emission spectrometry, using Ru as-is, and converting Ce, Zr, and Al to oxides. The methods for measuring the ruthenium dispersion degree and the crushing strength are the same as in Table 2.

Evaluation Result of Methanation Activity

The methanation activity of each of the catalysts F, G, H, and I was evaluated in the same manner as in Table 3. Table 6 shows the results.

[Table 6]

| Sample | $CO_2$ conversion rate (%) | |
|---|---|---|
| | 225°C | 250°C |
| Catalyst F | 24.9 | 42.0 |
| Catalyst G | 32.2 | 46.3 |
| Catalyst H | 31.0 | 44.5 |
| Catalyst I | 27.8 | 40.8 |

Evaluation of Examples 4 to 6 and Comparative Example 3

The results of the heat resistance evaluation will be considered next. The BET specific surface area of the ceria-supported alumina F after high-temperature firing was 46.3 $m^2$/g, which is a low value compared to those of the zirconia-supported aluminas A, B, and C (64.4 $m^2$/g, 56.3 $m^2$/g, and 61.3 $m^2$/g, respectively). Even if ceria (cerium oxide) is supported instead of zirconia, the heat resistance is improved, but it is clear that the effect is not as good as that in the case where zirconia is supported.

The BET specific surface areas of the ceria-zirconia-supported aluminas G and H after high-temperature firing were 52.9 $m^2$/g and 48.7 $m^2$/g, respectively. Although they are higher than the value of the ceria-supported alumina F (46.3 $m^2$/g), they are lower than the value of the zirconia-supported alumina B (56.3 $m^2$/g), and the higher the amount of ceria supported is, the lower the BET specific surface area after high-temperature firing is, and therefore it can be understood that the addition of ceria to alumina has a certain effect in improving the heat resistance, but the coexistence of ceria in zirconia-supported alumina reduces the heat resistance.

The zirconia-supported alumina I in which zirconia was supported using zirconium dichloride oxide dihydrate showed improved heat resistance as compared with the alumina E. However, compared with the zirconia-supported aluminas A, B, and C, the decrease in BET specific surface area after high-temperature firing was somewhat large, and the phase transformation to alpha-type also progressed, and therefore it can be understood that it is more preferable to use a solution of zirconium nitrate oxide acidified with nitric acid or an aqueous solution of zirconium acetate oxide in the supporting of the zirconium.

The dispersion degrees of ruthenium supported on the catalysts F, G, H, and I were 0.46, 0.51, 0.57, and 0.65, respectively. Compared to the value for the catalyst E using the alumina E (0.64), the value for the catalyst I improved slightly, but the values for the catalysts F, G, and H decreased, and therefore it can be understood that the coexistence of ceria reduces the degree of dispersion of ruthenium.

The crushing strength of the catalyst H improved compared to the catalyst E, but the crushing strengths of the catalysts F, G, and I were lower than that of the catalyst E.

The methanation activity of the catalysts F, G, H, and I was significantly higher than that of catalyst E, in which only ruthenium was supported on activated alumina. However, the methanation activity of the catalyst F is significantly lower than that of the catalysts B and C, and the superiority of the catalyst in which zirconia and ruthenium are supported on activated alumina of the present invention is clear. Also, when comparing the methanation activity of the catalysts B, G, and H, which have the same level of amounts of zirconia supported, it is understood that as the amount of ceria added increases, the methanation activity decreases. In the catalyst of the present invention, there is no problem in containing ceria, but from the viewpoint of heat resistance and methanation activity, it is preferable to use 5 parts by mass or less of ceria with respect to 100 parts by mass of activated alumina, and it is more preferable to use 1 part by mass or less of ceria.

Example 7

5.4 g of zirconium nitrate oxide dihydrate was dissolved in dilute nitric acid prepared by mixing 4.2 g of 60% nitric acid and 32 g of pure water, to obtain an aqueous solution in which a zirconium compound is dissolved. 50 g of activated alumina (Sumitomo Chemical Co., Ltd., KHA-24, 2 to 4 mm spherical molded body) was immersed in the above aqueous solution and impregnated for 15 hours to obtain an impregnated body. After the impregnated body was evaporated to dryness on a hot plate, it was dried in a dryer maintained at 125°C for 1.5 hours to obtain a dry body. This dry body was loaded into an electric furnace, heated from normal temperature to 700°C over 3 hours with a flow of air, and was kept at 700°C for 4 hours and fired. Thereafter, it was allowed to cool to normal temperature over 3 hours to obtain a zirconia-supported alumina J.

98 parts by mass of the zirconia-supported alumina J was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst J.

Example 8

10.8 g of zirconium nitrate oxide dihydrate was dissolved in dilute nitric acid prepared by mixing 11.5 g of 60% nitric acid and 35 g of pure water, to obtain an aqueous solution in which a zirconium compound is dissolved. 50 g of activated alumina (Sumitomo Chemical Co., Ltd., KHA-24, 2 to 4 mm spherical molded body) was immersed in the above aqueous solution and impregnated for 15 hours to obtain an impregnated body. After the impregnated body was evaporated to dryness on a hot plate, it was dried in a dryer maintained at 125°C for 1.5 hours to obtain a dry body. This dry body was loaded into an electric furnace, heated from normal temperature to 700°C over 3 hours with a flow of air, and was kept at 700°C for 4 hours and fired. Thereafter, it was allowed to cool to normal temperature over 3 hours to obtain a zirconia-supported alumina K.
98 parts by mass of the zirconia-supported alumina K was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst K.

Example 9

13.09 g of zirconium dichloride oxide dihydrate was dissolved in 22.5 g of pure water to obtain an aqueous solution in which a zirconium compound is dissolved. 50 g of the same activated alumina as used in Example 4 was immersed in the above aqueous solution and impregnated for 15 hours to obtain an impregnated body. The impregnated body was evaporated to dryness on a hot plate and then dried in a dryer maintained at 120°C for 1 hour to obtain a dry body. This dry body was loaded into an electric furnace, heated from normal temperature to 500°C over 3 hours with a flow of air, and was kept at 500°C for 2 hours and fired. Thereafter, it was allowed to cool to normal temperature over 3 hours to obtain a zirconia-supported alumina L.
98 parts by mass of the zirconia-supported alumina L was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst L.

Comparative Example 4

98 parts by mass of the same activated alumina (referred to as alumina M) as used in Example 7 was impregnated with an aqueous ruthenium chloride solution containing 2 parts by mass of ruthenium, dried at 80°C for 4 hours, immersed in a 0.375-N NaOH aqueous solution for 15 hours, subjected to liquid-phase reduction with a 0.3% aqueous hydrazine solution, and washed with hot water at 80°C, and then was dried at 80°C for 4 hours to obtain a catalyst M.

Heat Resistance Evaluation Result

The zirconia-supported aluminas J, K, and L, and the alumina M were each subjected to high-temperature firing at 1050°C for 6 hours in air. Table 7 shows the $ZrO_2$ content, BET specific surface area (before and after high-temperature firing), and degree of phase transformation to alpha-type of each sample.

[Table 7]

| Sample | $ZrO_2$ (wt%) | BET specific surface area ($m^2/g$) | Degree of transformation to $\alpha$ (%) |
|---|---|---|---|
| Zirconia-supported alumina J | 3.7 | 147 → 61.1 | 0 |
| Zirconia-supported alumina K | 6.8 | 141 → 67.6 | 0 |
| Zirconia-supported alumina L | 6.4 | 147 → 49.2 | 3.7 |
| Alumina M | - | 162 → 34.7 | 34 |
| (Note) The BET specific surface area is written in the format before high-temperature firing → after high-temperature firing. Also, the degree of phase transformation to alpha-type is the value after high-temperature firing. | | | |

Measuring Method

The $ZrO_2$ content of each sample of the zirconia-supported aluminas J, K, and L, and the alumina M was determined by performing acid decomposition, quantifying Zr through ICP emission spectrometry, and converting Zr to an oxide. The methods for measuring the BET specific surface area and the degree of phase transformation to alpha-type are the same as in Table 1.

Evaluation Results of Ru Dispersion Degree and Crushing Strength

The metal dispersion degree of the supported ruthenium and the crushing strength were evaluated for each of the catalysts J, K, L, and M. Table 8 shows the Ru, $ZrO_2$, and $Al_2O_3$ contents, BET specific surface area, ruthenium dispersion degree, and crushing strength of each catalyst.

[Table 8]

| Sample | Ru (wt%) | $ZrO_2$ (wt%) | $Al_2O_3$ (wt%) | Ru dispersion degree (-) | Crushing strength (N) |
|---|---|---|---|---|---|
| Catalyst J | 1.99 | 3.6 | 94.4 | 0.66 | 181 |
| Catalyst K | 1.91 | 6.2 | 91.9 | 0.72 | 180 |
| Catalyst L | 2.00 | 5.8 | 92.2 | 0.57 | 133 |
| Catalyst M | 1.89 | - | 98.1 | 0.60 | 139 |

Measuring Method

The contents of Ru, $ZrO_2$, and $Al_2O_3$ of each of the catalysts J, K, L, and M were obtained by performing acid decomposition, quantifying Ru, Zr, and Al through ICP emission spectrometry, using Ru as-is, and converting Zr and Al to oxides. The methods for measuring the ruthenium dispersion degree and the crushing strength are the same as in Table 2.

Evaluation Result of Methanation Activity

The methanation activity of each of the catalysts J, K, L, and M was evaluated in the same manner as in Table 3. Table 9 shows the results.

[Table 9]

| Sample | $CO_2$ conversion rate (%) | |
|---|---|---|
| | 225°C | 250°C |
| Catalyst J | 43.7 | 63.0 |
| Catalyst K | 45.2 | 63.8 |
| Catalyst L | 35.7 | 54.3 |
| Catalyst M | 17.2 | 37.2 |

Electron Probe Microanalysis Results

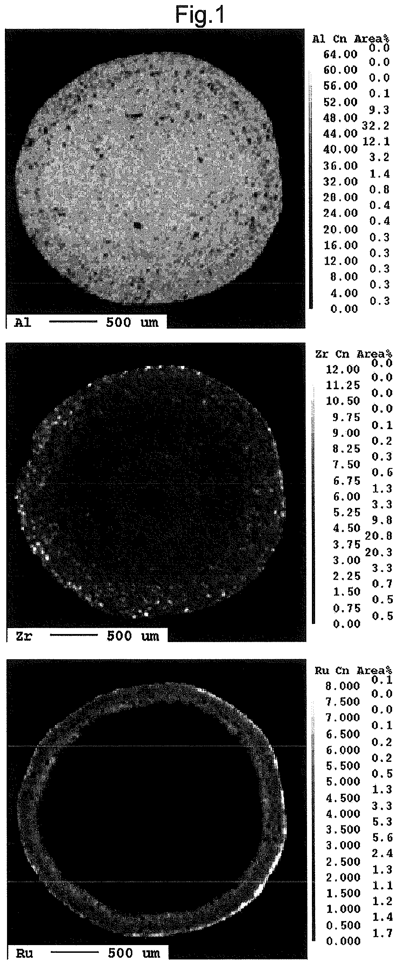

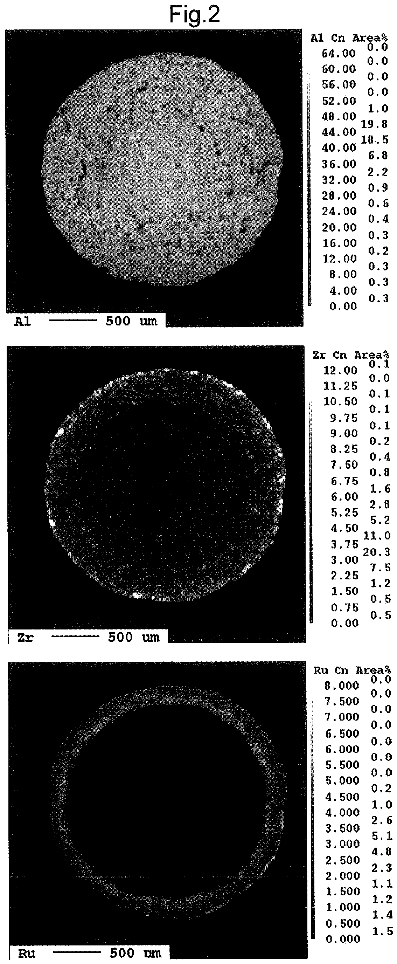

For the catalyst K, the distributions of ruthenium and zirconia in the catalyst molded body were studied through electron probe microanalysis. The measurement was performed using a field emission electron probe microanalyzer JXA-8500F manufactured by JEOL Ltd., and analysis was performed under the following analysis conditions. Accelerating voltage: 15 kV, irradiation current: 500 nA, analysis range: 3.125 mm × 3.125 mm, target elements and detected characteristic X-rays: Al (Kβ), Zr (Lα), Ru (Lα). FIGS. 1 and 2 show the measurement results for two different molded body particles of the catalyst K. The measurement results are expressed in terms of mass% of each element. Note that the measurement results are shown in the table in terms of mass% of each element. For example, when describing Al, the measurement results are color-coded in increments of 4 mass%, such as over 0 mass% and 4 mass% or less, over 4 mass% and 8 mass% or less, and over 8 mass% and 12 mass% or less, and the measurement results are shown in grayscale such that the higher the mass-basedAl content (Al Cn) is, the lighter the gray color is. The Area% column shows the surface area ratio of the region of the content range in the entire field of view. Note that since the edge of the field of view includes a portion where no catalyst exists, even if the values shown in the Area% column are added together, they do not add up to 100%.

As is clear from FIGS. 1 and 2, in both molded body particles, ruthenium is supported in an eggshell-like form in which ruthenium is concentrated in a range of about 0.3 mm from the surface. In contrast, the zirconia is supported up to the center, but the amount of zirconia supported is higher in the shell region where the ruthenium is supported.

Based on the measurement results of FIG. 1, when the mass% of the Al elements is converted into the mass% of $Al_2O_3$ and the amount of Ru supported per 100 parts by mass of alumina ($Al_2O_3$) is calculated, 4 parts by mass of ruthenium per 100 parts by mass of alumina are supported on average in the region of 0.3 mm from the molded body particle surface, and almost no ruthenium is supported toward the center (0.1 parts by mass or less on average).

Also, based on the measurement results in FIG. 1, when the mass% of the Al elements is converted into the mass% of $Al_2O_3$, the mass% of the Zr elements is converted into the mass% of $ZrO_2$, and the amount of zirconia ($ZrO_2$) supported per 100 parts by mass of alumina ($Al_2O_3$) is calculated, 9 parts by mass of zirconia per 100 parts by mass of alumina is supported on average in the range of 0.3 mm from the surface of the molded body particles, and 5 parts by mass of zirconia per 100 parts by mass of alumina is supported on average toward the center. Based on the analysis results shown in Table 8, the amount of zirconia supported by the catalyst K is 7 parts by mass per 100 parts by mass of alumina, and therefore in the center portion, the supported amount is about 70% of the molded body particle average, and in the shell portion, the supported amount is about 130% of the molded body particle average.

X-Ray Diffraction Measurement Result

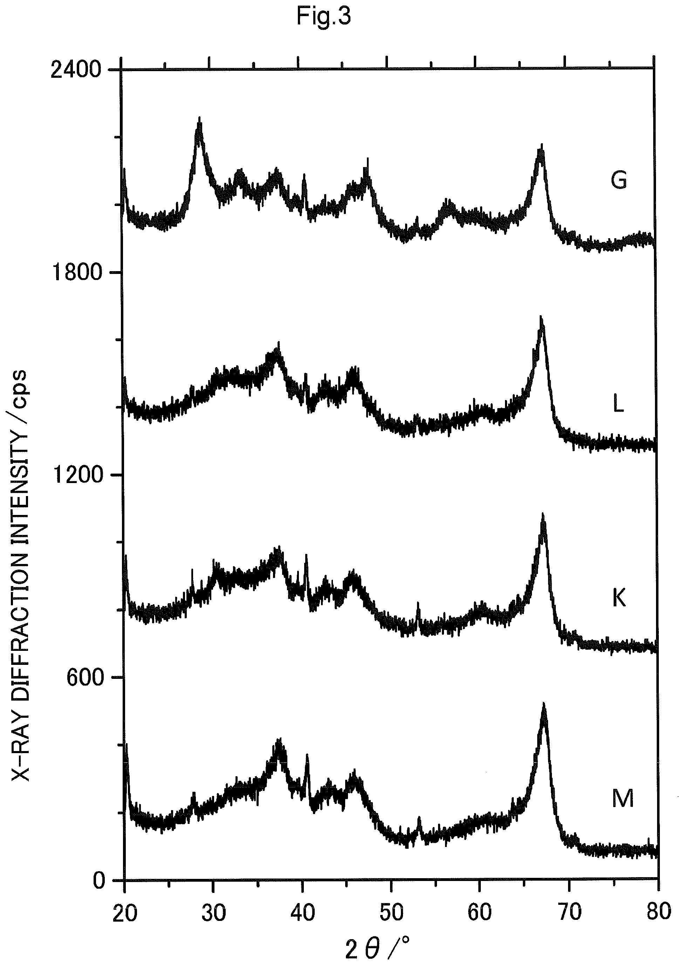

X-ray diffraction measurement was performed on the catalysts K, L, M, and G. The measurement was performed under the following conditions using an X-ray diffractometer (XRD-6100 manufactured by Shimadzu Corporation) equipped with a graphite monochromator. X-ray source: Cu-Ka rays (0.1542 nm) emitted from an X-ray tube (Cu target, tube voltage 40 kV, tube current 40 mA). Measurement conditions: Step scan method, 0.02° steps, cumulative time at each step: 1.2 seconds, detection slit: 0.15 mm. The X-ray diffraction pattern of each sample was as shown in FIG. 3.

With the catalyst K, diffraction lines not seen with the catalyst M were observed at 30.3° ($\pm$0.5°), 50.5° ($\pm$0.5°), and the like. These are the diffraction lines of tetragonal zirconia. On the other hand, the catalyst L contains zirconia in the same manner as the catalyst K, and the amount of zirconia supported is also similar, but the diffraction lines of tetragonal zirconia were not clearly observed. Based on the fact that the methanation activity of the catalyst K was significantly higher than that of the catalyst L and the result of X-ray diffraction measurement, it can be said that containing tetragonal zirconia imparts high methanation activity.

In the X-ray diffraction pattern of the catalyst G, diffraction lines not seen with the catalyst M were observed near 28.8° and 48.0°. These diffraction lines are attributed to a solid solution of cerium oxide and zirconium oxide (zirconia). That is, in the catalyst G, a solid solution is formed with supported zirconium oxide and cerium oxide, and zirconia alone does not exist. When cerium was further added to the catalyst in which ruthenium and zirconia were supported on activated alumina, as the amount of cerium added increased, the methanation activity decreased and the heat resistance decreased, and it is presumed that the reason for this is that the original effect of zirconia is no longer exhibited due to forming a solid solution with zirconium oxide and cerium oxide.

Evaluation Result of Heat Resistance of Catalyst

The catalysts K, L, and M were subjected to high-temperature firing at 1050°C for 6 hours in air, and the degree of phase transformation to alpha-type was calculated using the same method as shown in Table 1. The degrees of phase transformation to alpha-type of the catalysts K, L, and M after high-temperature firing were 4.0%, 26%, and 64%, respectively. Compared with the degree of phase transformation to alpha-type after high-temperature firing of the zirconia-supported aluminas K and L, and the alumina M, all of them are slightly higher, and it can be understood that the phase transformation to alpha-type progresses more easily after supporting ruthenium, but it is clear that the heat resistance is improved by supporting zirconia, and it is also clear that the heat resistance of the catalyst K supporting zirconia under acidic conditions with nitric acid is particularly excellent.

Results of Water Vapor Resistance Evaluation

For the catalysts K and M, the stability of alumina under high partial water vapor pressure was evaluated by allowing a gas consisting of 0.5 MPa (absolute pressure) water vapor and 0.1 MPa (absolute pressure) nitrogen to flow therethrough at 700°C. The BET specific surface area was measured, and the degree of phase transformation to alpha-type and crushing strength were measured through X-ray diffraction for samples treated for predetermined amounts of time. The measurement of the BET specific surface area and the measurement of the degree of phase transformation to alpha-type through X-ray diffraction were performed in the same manner as in Table 1, and measurement of the crushing strength was performed in the same manner as in Table 2. Table 10 shows the results.

[Table 10]

| Sample | Treatment time (h) | BET specific surface area ($m^2$/g) | Degree of transformation to $\alpha$ (%) | Strength (N) |
|---|---|---|---|---|
| Catalyst K | 0 | 144 | 0 | 180 |
| | 20 | 83.0 | 0 | 81.9 |
| | 100 | 71.1 | 0 | 52.5 |
| | 200 | 61.1 | 5.9 | 45.3 |
| Catalyst M | 0 | 183 | 0 | 139 |
| | 20 | 87.8 | 0 | 41.2 |
| | 100 | 47.8 | 39 | 14.5 |
| | 200 (grain) | 1.72 | 83 | Unmeasurable |
| | 200 (powder) | 2.31 | 77 | Unmeasurable |
| (Note) The catalyst M did not maintain its original shape after 200 hours of treatment, and therefore it was analyzed by dividing it into a granular portion and a powdery portion. | | | | |

The phase transformation to alpha-type of the catalyst M progressed until it lost its original shape after 200 hours, whereas the phase transformation to alpha-type of the catalyst K did not progress much even after 200 hours, and the catalyst K maintained a constant BET specific surface area and strength. The degree of phase transformation to alpha-type after treatment under high partial water vapor pressure corresponds to the degree of phase transformation to alpha-type after high-temperature (1050°C) firing, and it can be understood that the treatment for firing at 1050°C for 6 hours in air is equivalent to treatment for 100 to 200 hours under high partial water vapor pressure at 700°C. Phase transformation to alpha-type progresses over time under conditions of high partial water vapor pressure even at lower temperatures, for example, about 500°C, but since phase transformation to alpha-type is not likely to progress in the catalyst of the present invention even under such conditions, the catalyst of the present invention can be used stably for a long time.

Based on the above results, it is clear that the methanation catalyst molded body of the present invention has high activity at low temperatures, sufficient strength for industrial use, and heat resistance under high temperature and high water vapor pressure conditions.

Note that the configurations disclosed in the above embodiments (including other embodiments, the same applies hereinafter) can be applied in combination with configurations disclosed in other embodiments as long as there is no contradiction, the embodiments disclosed in this specification are exemplary, and the embodiments of the present invention are not limited thereto, and can be modified as appropriate without departing from the object of the present invention.

Industrial Applicability

The present invention can be used, for example, as a catalyst for producing a fuel gas containing methane as a main component and can be used as city gas, by reacting carbon dioxide and hydrogen.

**Claims**

1.  A carbon dioxide methanation catalyst molded body comprising:

    an activated alumina molded body; and
    zirconia and ruthenium supported on the activated alumina molded body
    wherein the amount of zirconia supported is 3 to 10 parts by mass with respect to 100 parts by mass of the activated alumina molded body, the amount of ruthenium supported is 0.1 to 5 parts by mass with respect to 100 parts by mass of the activated alumina molded body and the carbon dioxide methanation catalyst molded body is a molded body having a particle diameter of 2 to 20 mm.

2. The carbon dioxide methanation catalyst molded body according to claim 1, wherein the ruthenium is supported on the activated alumina molded body in an eggshell shape having a shell portion with a thickness of 0.2 to 0.4 mm, the zirconia is supported in a support amount of 50% or more and less than 100% in a center portion using the average amount of zirconia supported in the entire activated alumina molded body as a reference, and the amount of zirconia supported is higher than 100% in the shell portion where the ruthenium is supported.

3. The carbon dioxide methanation catalyst molded body according to claim 1 or 2, wherein the zirconia is present mainly as a tetragonal crystal.

4. The carbon dioxide methanation catalyst molded body according to any one of claims 1 to 3, wherein the content of cerium oxide is 5 parts by mass or less with respect to 100 parts by mass of the activated alumina molded body.

5. The carbon dioxide methanation catalyst molded body according to any one of claims 1 to 4, wherein a degree of phase transformation to alpha-type, which is measured through a procedure of firing the activated alumina molded body supporting the zirconia at 1050°C for 6 hours in air, and then measuring the degree of phase transformation to alpha-type through X-ray diffraction measurement using Cu-Ka rays as a radiation source, is 10% or less.

6. The carbon dioxide methanation catalyst molded body according to any one of claims 1 to 4, wherein a degree of phase transformation to alpha-type, which is measured through a process of firing at 1050°C for 6 hours in air, and then measuring the degree of phase transformation to alpha-type through X-ray diffraction measurement using Cu-Ka rays as a radiation source, is 10% or less.

7. A method for producing a carbon dioxide methanation catalyst molded body, comprising:

a zirconium impregnation step of impregnating an activated alumina molded body having a particle diameter of 2 to 20 mm with an aqueous solution in which a water-soluble compound of zirconium is dissolved, to obtain a zirconium impregnated body;
a drying step of drying the zirconium-impregnated body to obtain a dry body;
a firing step of firing the dry body at 500 to 800°C in air to obtain activated alumina in which zirconia is supported in a dispersed manner;
a ruthenium impregnation step of impregnating the activated alumina in which the zirconia is supported in a dispersed manner with an aqueous solution in which a water-soluble compound of ruthenium is dissolved, to obtain a ruthenium impregnated body; and
a ruthenium immobilization step of immobilizing the ruthenium by drying the ruthenium impregnated body.

8. The method for producing a carbon dioxide methanation catalyst molded body according to claim 7, wherein the zirconium impregnation step is performed using an aqueous solution in which a water-soluble compound of zirconium is dissolved and that is acidified with nitric acid.

# Fig.1

## Fig.2

## Fig.3

## INTERNATIONAL SEARCH REPORT

| | International application No. |
|---|---|
| | **PCT/JP2022/018308** |

**A. CLASSIFICATION OF SUBJECT MATTER**

*B01J 23/46*(2006.01)i; *B01J 23/63*(2006.01)i; *B01J 37/02*(2006.01)i; *B01J 37/04*(2006.01)i; *B01J 37/08*(2006.01)i; *C07B 61/00*(2006.01)i; *C07C 1/12*(2006.01)i; *C07C 9/04*(2006.01)i
FI: B01J23/46 301M; B01J23/63 M; B01J37/02 101D; B01J37/04 102; B01J37/08; C07B61/00 300; C07C1/12; C07C9/04

According to International Patent Classification (IPC) or to both national classification and IPC

**B. FIELDS SEARCHED**

Minimum documentation searched (classification system followed by classification symbols)

B01J21/00-38/74; C07B61/00; C07C1/12, 9/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 07-116516 A (IDEMITSU KOSAN CO LTD) 09 May 1995 (1995-05-09)<br>examples 1, 3, claims, paragraphs [0001], [0012]-[0013], [0027], [0037]-[0058], [0090] | 1, 4-6 |
| Y | JP 11-179204 A (COSMO SOGO KENKYUSHO KK) 06 July 1999 (1999-07-06)<br>claims, paragraphs [0001]-[0017], [0081], examples | 1, 4-6 |
| Y | JP 2019-076862 A (TOYOTA CENTRAL RES & DEV) 23 May 2019 (2019-05-23)<br>claims, paragraphs [0001]-[0017], [0060], examples | 1, 4-6 |
| Y | CN 106268858 A (SHANXI INSTITUTE OF COAL CHEMISTRY, CHINESE ACADEMY OF SCIENCES) 04 January 2017 (2017-01-04)<br>claims, paragraphs [0001]-[0033], examples | 1, 4-6 |
| A | WO 2005/079978 A1 (IDEMITSU KOSAN CO LTD) 01 September 2005 (2005-09-01)<br>entire text, all drawings | 1-8 |
| A | JP 2006-239551 A (MITSUBISHI HEAVY IND LTD) 14 September 2006 (2006-09-14)<br>entire text, all drawings | 1-8 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **16 June 2022** | **28 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

International application No.

**PCT/JP2022/018308**

| Patent document cited in search report | | | Publication date (day/month/year) | Patent family member(s) | Publication date (day/month/year) |
|---|---|---|---|---|---|
| JP | 07-116516 | A | 09 May 1995 | (Family: none) | |
| JP | 11-179204 | A | 06 July 1999 | (Family: none) | |
| JP | 2019-076862 | A | 23 May 2019 | (Family: none) | |
| CN | 106268858 | A | 04 January 2017 | (Family: none) | |
| WO | 2005/079978 | A1 | 01 September 2005 | US 2007/0172416 A1<br>entire text, all drawings<br>EP 1852181 A1<br>CA 2555427 A<br>CN 1921937 A<br>KR 10-2007-0001141 A<br>TW 200534920 A | |
| JP | 2006-239551 | A | 14 September 2006 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S60235893 A
- JP 2015124217 A
- JP 2018135283 A
- JP 2009131835 A
- JP 2019048249 A
- JP 2019076862 A
- JP S574232 A
- JP S5024200 A
- JP 2020132514 A
- JP 2011513055 A
- JP H7116516 A

**Non-patent literature cited in the description**

- Chemical Process Collection. 1970, 153
- Catalysis Handbook. 2008, 535
- **KAWAGOE ; MATSUDA ; MATSUSHIMA ; UEMATSU.** *Hitachi Hyoron,* 1986, vol. 68 (10), 73
- **E.I. KOYTSOUMPA ; S. KARELLAS.** *Renewable and Sustainable Energy Reviews,* 2018, vol. 94, 536